# FASCICULE DE BREVET EUROPEEN

(11) **EP 3 378 431 B1**
(45) Date de publication et mention de la délivrance du brevet: **27.12.2023**
(21) Numéro de dépôt: 18171878.4
(22) Date de dépôt: 08.11.2012
(51) Int. Cl.: A61B 34/30, A61B 17/00

(54) **DISPOSITIF ASSISTÉ PAR ROBOTIQUE DE POSITIONNEMENT D'INSTRUMENT CHIRURGICAL PAR RAPPORT AU CORPS D'UN PATIENT**
ROBOTERUNTERSTÜTZTE VORRICHTUNG ZUR POSITIONIERUNG EINES CHIRURGISCHEN INSTRUMENTS BEZÜGLICH DES KÖRPERS EINES PATIENTEN
ROBOTIC DEVICE FOR POSITIONING A SURGICAL INSTRUMENT RELATIVE TO THE BODY OF A PATIENT

(30) Priorité: 30.11.2011 FR 1160974
(43) Date de publication de la demande: 26.09.2018
(62) Demande divisionnaire de: 12795507.8
(73) Titulaire: Medtech, 34170 Castelnau le Lez (FR)
(72) Inventeur: MAILLET, Pierre, 34130 Saint-Aunes (FR); NAHUM, Bertin, 34670 Baillargues (FR); BADANO, Fernand, 69100 Villeurbanne (FR); DEHOUR, Patrick, 30260 Crespian (FR)
(74) Mandataire: Taor, Simon Edward William

(56) Documents cités:
- WO-A2-2007/002926
- DE-A1-102008 022 924
- US-A1- 2006 142 657
- US-A1- 2008 033 410

## Description

La présente invention entre dans le domaine médical, en particulier dans les dispositifs destinés à la méthodologie opératoire lors de la préparation et la réalisation d'interventions chirurgicales.

L'invention concerne spécifiquement des dispositifs utilisés avec l'imagerie médicale anatomique, dans le but d'effectuer des interventions chirurgicales assistées par la robotique.

La présente invention trouvera une application préférentielle, mais aucunement limitative, dans des dispositifs pour des interventions chirurgicales de la zone anatomique du rachis.

Pour ce faire, l'invention concerne un dispositif de positionnement d'instrument chirurgical par rapport au corps d'un patient.

On notera que l'invention sera décrite selon un exemple particulier d'intervention au niveau du rachis lombaire ou lombal, au niveau de la courbure antérieure lordose de la colonne vertébrale. Toutefois, l'invention pourra être utilisée par une intervention au niveau des rachis cervicaux supérieur et inférieur, du rachis dorsal ou thoracique, ainsi que du rachis sacré et du coccyx.

Dans cette zone anatomique, les actes chirurgicaux sont des opérations délicates, minutieuses et à risques, qui nécessitent de percer, casser ou fraiser une vertèbre, de disséquer des muscles, des ligaments, des disques vertébraux avec précision, tout en évitant d'endommager la moelle épinière, les racines nerveuses, les veines et les nerfs.

A titre d'exemple, les actes couramment pratiqués comprennent la laminectomie, la libération radiculaire, dans les cas d'un canal lombaire étroit, ou d'une hernie discale ; l'arthrodèse consistant à fusionner des vertèbres par vissages pédiculaires, la kyphoplastie et la vertébroplastie consistant à injecter un ciment au sein du corps vertébral.

De telles opérations sont mises en œuvre via un canal opératoire le plus étroit possible, afin de diminuer l'hospitalisation et faciliter la récupération du patient, plus encore au travers d'une intervention mini-invasive ou percutanée. L'étroitesse de ce canal opératoire rend difficile la précision de l'intervention du praticien, la visibilité diminuant d'autant en raison des saignements. En l'état, il en résulte un taux de mauvais placement de vis pédiculaires jusqu'à 25 %, sachant que les mauvais placements agressifs représentent 3 à 5%.

Afin d'améliorer la précision de son intervention, le chirurgien a désormais recours à des systèmes d'imagerie médicale anatomique, en particulier la fluoroscopie et la navigation.

Tout d'abord, la fluoroscopie génère des rayons X sur une période permettant d'acquérir des images en continue et ainsi d'obtenir une vision directe où le chirurgien peut suivre en temps réel la progression de son outil au sein de la structure anatomique. Toutefois, l'exposition prolongée ou répétitive à ces radiations ionisantes étant nocive pour le personnel médical comme pour le patient, l'utilisation de cette technique est donc volontairement limitée.

D'autre part, la navigation permet de visualiser un outil virtuel sur une imagerie pré ou per opératoire, sur laquelle le chirurgien constate en temps réel la progression de son outil, encore davantage dans le cas d'une navigation en trois dimensions (3D).

Toutefois, un premier inconvénient provient de la complexité des calculs de positionnement des outils chirurgicaux, amenant à des approximations et entraînant de mauvais positionnement. De plus, les techniques existantes nécessitent le positionnement d'un marqueur fixe sur le corps du patient, actuellement vissé de manière invasive dans l'épine dorsale par exemple et sur lequel est dirigée l'optique d'acquisition d'images du système de navigation.

En outre, ces techniques ne permettent pas de s'affranchir des erreurs liées à la pratique manuelle de l'acte chirurgical par le praticien, en particulier dans le cas d'une étape contraignante de perçage, puis de vissage dans un pédicule.

C'est pour cette raison que se sont développés des systèmes robotiques d'aide à la chirurgie, qui permettent d'assister le chirurgien et d'assurer une précision et une répétitivité du geste opératoire de façon mécanique.

Dans ce cadre, un problème supplémentaire lors d'une opération réside dans la gestion des mouvements anatomiques du patient consécutifs à sa propre respiration, ainsi qu'aux battements de son coeur. En particulier, la respiration dépend de l'activité du diaphragme générant les mouvements thoraciques et pulmonaires contributifs aux échanges gazeux. Cette activité musculaire entraine une déformation des parties anatomiques collatérales, comme l'abdomen et le rachis. L'amplitude de cette déformation dépend de la ventilation minute (VM), en fonction de son volume et de sa fréquence, mais aussi de la position du patient, à savoir débout, assis ou bien allongé sur le ventre, sur le dos ou encore de côté.

Dans le cas d'une intervention sur le rachis, ce dernier se déplace d'une façon plus conséquente pour les vertèbres thoraciques et dans une moindre mesure pour les vertèbres lombaires. De plus, les mouvements d'une vertèbre spécifique peuvent être modifiés par l'action du chirurgien dans le cadre de son intervention, notamment lorsqu'il perce ou casse la structure osseuse, ou bien coupe des muscles et ligaments qui soutiennent également la structure vertébrale.

Afin de limiter ces mouvements lors d'une intervention lombaire, quand la voie d'accès le permet, le patient est allongé sur le ventre, en prenant la précaution de laisser libre les mouvements du ventre sous le niveau du bassin pectoral. Le patient est alors immobilisé dans cette position par des mécanismes et accessoires de la table d'opération. Ce décubitus ventral particulier permet de diminuer considérablement l'amplitude des mouvements du rachis lombaire.

Néanmoins, la respiration et surtout les efforts extérieurs provenant de l'intervention génèrent des mouvements réciproquement périodiques et impromptus du rachis lombaire de plusieurs millimètres, que le chirurgien est alors obligé de compenser par sa dextérité et son acuité visuelle.

Dans le cas d'une opération assistée par la robotique, il est alors primordial de mesurer ces mouvements pour que le bras robotisé s'adapte automatiquement à ces mouvements, afin de conserver l'amélioration de la précision robotique par rapport à celle du chirurgien, tout en accompagnant lesdits mouvements avec une rapidité d'exécution correspondant la vélocité de la cible.

Pour ce faire, pour être en phase avec la planification préopératoire sur l'imagerie 3D, les mouvements de la cible anatomique, à savoir la vertèbre lombaire, doivent être mesurés et compensés en temps réel pour conserver la précision millimétrique du dispositif d'aide au repérage.

A l'heure actuelle, la solution du marqueur vissé dans l'épine dorsale se pratique, notamment en navigation. Un algorithme calcule alors les compensations dans le repère de l'image qui est visualisée et qui sont transmises en temps réel au bras robotisé. Toutefois, cette solution présente toujours l'inconvénient d'être invasive. De plus, la reconnaissance optique du marqueur présente des risques d'être masqué par le praticien qui, répercutés audit calcul, génère des différences de déplacement du bras robotisé par rapport aux mouvements anatomiques ainsi captés.

Une telle solution est décrite en partie dans le document US 2006/142657, ayant pour objet un système comprenant un premier bras robotisé et un second bras passif. Ledit premier bras porte un instrument, notamment chirurgical, tandis que le second bras est rendu solidaire à son extrémité distale d'un marqueur ancré dans l'os de la zone anatomique visée. Ainsi, le mouvement de l'os induit le déplacement du marqueur qui est capté par le bras passif, puis répercuté au bras robotisé.

Toutefois, sans imagerie, cette solution se limite à l'opération de l'os en lui-même et ne permet pas de compenser des mouvements des autres tissus. En somme, c'est l'ancrage mécanique dans l'os qui permet suivre les mouvements de la zone.

Une solution non invasive est imaginée au travers du document WO 2007/002926, décrivant un système robotisé d'irradiation de tissus pour traiter le cancer. En particulier, une source d'émission de radiation, « LINAC », est portée par un premier bras robotisé. Un second bras sert uniquement de support à une sonde à ultrasons, positionnée au niveau de la zone anatomique à traiter et enregistrant des images de ladite zone. Ces images servent à calculer les déplacements internes de la zone anatomique.

Toutefois, la position dans l'espace de la sonde est déterminée par un système optique distinct et supplémentaire, entraînant toujours les problèmes susmentionnés.

Ce document prévoit aussi d'utiliser des marqueurs placés au niveau de la zone anatomique, directement collés sur la peau. La détection des déplacements de ces marqueurs permet de déterminer les mouvements externes de la zone anatomique.

De plus, un traitement sur la base d'une modélisation mathématique est nécessaire pour obtenir les coordonnées relatives pour la correction de la trajectoire du LINAC, par rapport aux déplacements internes et externes réellement mesurés de la zone anatomique. Ce calcul provoque un temps de traitement non négligeable, rendant difficile l'application dans le cadre d'une intervention sur une zone anatomique comme le rachis, et générant toujours des degrés d'erreurs.

Une autre solution est décrite dans le document DE 10 2008 022924. Un système d'imagerie porté par un bras robotisé dit « C-arm » est capable d'effectuer une succession de clichés qui, après traitement, permettront de visualiser le mouvement de la zone anatomique ciblée.

Toutefois, le bras C-arm sert uniquement de support et pour positionner ledit système d'imagerie, en fonction de l'orientation souhaitée des clichés à prendre. Ce bras n'est nullement asservi, pour suivre ladite zone anatomique en fonction de ses mouvements. En somme, une fois positionné, ce bras reste statique ou sa position est modifiée, sans tenir compte des mouvements de la cible.

Par ailleurs, le suivi des mouvements de la zone est obtenu par des marqueurs, sous forme de pastilles, collés sur la partie externe de la zone anatomique. Encore une fois, un système distinct optique ou électromagnétique permet de capter les déplacements desdits marqueurs et d'en déduire les mouvements externes de ladite zone anatomique. Cette déduction s'effectue par un traitement mathématique pour diriger un autre robot, porteur d'un instrument, notamment chirurgical.

Un autre exemple de solution connexe est mentionné dans le document US 2008/033410, qui décrit un unique bras robotisé et un support fixe, réglé initialement, sans déplacement possible en fonction de mouvements captés. En effet, ledit support comprend des tubes reliés à leurs extrémités par des noix de serrage, les maintenant solidaires et fixes les uns par rapport aux autres. Pour régler leurs positionnements relatifs, il est alors nécessaire de desserrer lesdites noix, modifier l'orientation puis de resserrer les noix.

En outre, encore une fois, ce système fait appel à une visualisation par un système complémentaire optique, peu précis.

L'invention a pour but de pallier les inconvénients de l'état de la technique en proposant un dispositif de positionnement d'instrument chirurgical par rapport au corps d'un patient faisant intervenir au moins un premier bras robotisé supportant au moins un instrument chirurgical et un second bras traqueur, robotisé lui aussi. Ce second bras robotisé a pour but de détecter en temps réel avec précision les mouvements dudit corps et les communiquer audit premier bras afin qu'il compense son positionnement en conséquence. En particulier, ledit second bras permet de détecter les mouvements internes et externes de la zone anatomique à traiter.

D'une part, la détection des mouvements internes est effectuée par des capteurs adaptés, notamment par l'intermédiaire d'une sonde à ultrasons positionnée au contact de la peau du patient en vis-à-vis de la zone anatomique visée. A partir des mouvements internes ainsi captés en temps réel, il est possible de répercuter ces déplacements internes vers ledit premier bras robotisé afin de compenser sa trajectoire.

A ce titre, on notera que le positionnement du premier bras robotisé s'effectue dans une phase d'initialisation au moyen d'un recalage d'une image de la zone acquise en per-opération par rapport à une imagerie acquise préalablement. Dès lors, il est possible de modifier ce recalage, en fonction des mouvements internes détectés pour les capteurs portés par le second bras.

D'autre part, le second bras se déplace sous l'action des déplacements de la zone anatomique, étant en liaison avec elle, de préférence par contact. Ce sont ces déplacements externes de la zone, induits audit second bras, qui peuvent alors être répercutés audit premier bras. En somme, le second bras bouge puisqu'il est contre la zone et il est possible, du fait qu'il est robotisé, de connaître automatiquement et avec une extrême précision ces déplacements pour les transmettre audit premier bras.

D'autre part encore, ledit second bras étant robotisé, sa position est parfaitement connue. Comme il porte les capteurs, leur position est alors elle aussi connue avec exactitude. Ainsi, il est possible de s'affranchir d'un système supplémentaire de détection, notamment optique, de la position des capteurs qui sont directement solidaires de l'extrémité dudit second bras.

Selon un aspect de l'invention, il est proposé un dispositif d'assistance chirurgicale robotique automatisé tel que décrit dans la revendication 1. Des caractéristiques optionnelles sont décrites dans les revendications dépendantes.

Ainsi, l'invention met en oeuvre une solution robotique d'assistance au guidage d'instruments chirurgicaux, de recalages anatomiques et de planification des gestes opératoires pour des chirurgies à ciel ouvert, mini-invasives et percutanées, offrant une mesure en temps réel des mouvements de la zone visée par l'opération, notamment une vertèbre lombaire, et compensant avec précision lesdits mouvements mesurés, intérieurement et extérieurement, pour positionner le bras robotisé chirurgien en conséquence, tout en corrigeant ses trajectoires robotiques et en conservant l'exactitude du guidage et le bon positionnement des instruments.

Par ailleurs, on notera que ledit second bras peut alors consister en des moyens de référencement des positions dans l'espace desdits capteurs de détection et que les moyens d'asservissement du positionnement dudit bras comprennent des moyens de prise en compte du référencement desdites positions.

En outre, lesdits moyens de recalage peuvent comprendre des moyens d'acquisition par registration percutanée par ultrasons ou par repérage de la structure interne de ladite zone anatomique par fluoroscopie.

Avantageusement, ledit dispositif peut comprendre des moyens de mesure des efforts d'au moins un desdits bras lors du contact avec ladite zone anatomique.

Préférentiellement, lesdits moyens de détection peuvent comprendre des capteurs à ultrasons pour détecter lesdits mouvements en temps réel par mesure percutanée, notamment au moyen de capteurs à ultrasons.

En particulier, les moyens de détection peuvent comprendre au moins un marqueur rendu solidaire de ladite zone anatomique, de manière à mesurer ses déplacements dans l'espace.

Enfin, ledit marqueur peut être un marqueur émettant un signal optique, ultrason, électromagnétique et/ou mécanique, tandis que les moyens de détection peuvent comprendre une réception dudit signal par ledit capteur réciproquement optique, ultrason, électromagnétique et/ou mécanique. Dès lors, la mesure des déplacements s'effectue par émission par ledit marqueur dudit signal, puis réception de ce signal par lesdits capteurs.

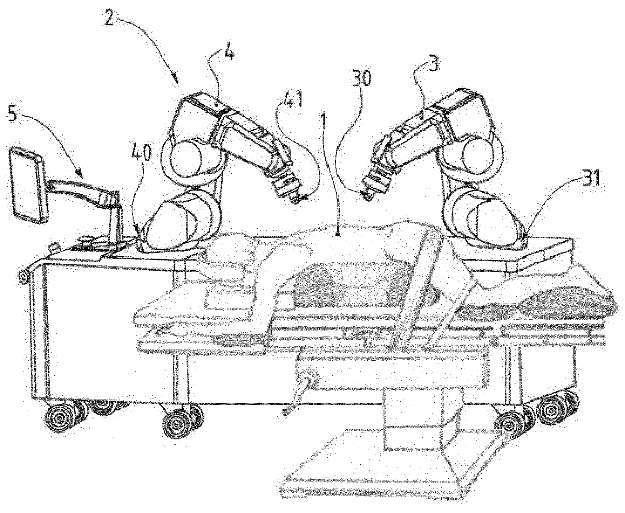

D'autres caractéristiques et avantages de l'invention ressortiront de la description détaillée qui va suivre des modes de réalisation non limitatifs de l'invention, en référence à l'unique figure annexée représentant schématiquement le dispositif robotisé en vue d'une intervention sur un patient allongé sur une table d'opération en décubitus ventral.

La présente invention concerne un dispositif pour le positionnement d' outil chirurgical assisté par robotique par rapport au corps 1 d'un patient et la correction d'un tel positionnement par rapport aux mouvements dudit corps 1.

L'invention sera décrite à titre d'exemple en référence à une zone anatomique dudit corps 1, notamment le rachis et plus particulièrement l'intervention sur une vertèbre lombaire.

Ainsi, l'invention concerne un dispositif 2 de mise en oeuvre en vue d'assurer un positionnement avec précision d'au moins un instrument chirurgical par rapport au corps 1 d'un patient, en particulier d'une zone anatomique déterminée.

Tout d'abord, dans la mise en œuvre du dispositif selon l'invention, on rend solidaire au moins un instrument chirurgical de l'extrémité 30 d'au moins un premier bras robotisé 3, de manière à maintenir, déplacer, positionner et guider ledit instrument chirurgical. Plusieurs bras peuvent chacun soutenir un ou plusieurs instruments.

En particulier, ledit premier bras 3 robotisé comprend plusieurs sections motorisées et articulées entre elles de manière à se déplacer dans l'espace. Ledit premier bras 3 robotisé présente une extrémité fixe 31, servant de base et définissant le repère tridimensionnel au sein duquel il évolue. L'extrémité distale opposée 30 présente des moyens adaptés pour recevoir en fixation amovible un ou plusieurs instruments chirurgicaux. Cette extrémité distale 30 peut aussi comprendre des moyens de réception de capteurs ou d'appareils de mesure.

Ainsi, le premier bras robotisé 3 remplace ou guide la main du praticien et maintient les outils chirurgicaux.

Avantageusement, on positionne par rapport audit corps 1 ledit premier bras 3 par recalage anatomique. Ce dernier s'effectue par recalage d'une image d'une zone de l'anatomie dudit patient acquise en per-opératoire par rapport à une imagerie acquise préalablement.

A ce titre, plusieurs techniques de recalage peuvent être utilisées. Elles consistent à collecter au moment ou lors de l'intervention des images de la zone anatomique du patient afin de les situer avec exactitude dans l'espace et le repère des instruments chirurgicaux portés par le premier bras 3. Pour ce faire, les images acquises sont comparées et mises en correspondance avec des images acquises au préalable, en mode préopératoire. C'est à partir de ces images préopératoires que le positionnement et le déplacement du premier bras robotisé 3 est configuré, afin de réaliser les gestes chirurgicaux se déroulant pendant l'intervention.

On notera que le recalage peut être effectué sur des images en deux ou trois dimensions, au moyen d'un logiciel informatique.

Un exemple de méthodologie de recalage anatomique est décrit en détail dans le document FR 2 917 598 et permet de capter au sein d'un espace tridimensionnel la forme d'une surface anatomique d'une zone d'intérêt du corps d'un patient. Par la suite, la forme mesurée en per-opératoire par le robot sera recalée sur l'image d'un scanner réalisé préalablement en préopératoire, notamment par un scanner de type Imagerie à Résonance Magnétique (« IRM »). L'opération d'enregistrement surfacique est effectuée soit par un palpeur mécanique porté par le bras robotisé, venant au contact de ladite zone anatomique, pour enregistrer dans le repère tridimensionnel du robot les positions des points palpés, soit par un télémètre laser sans contact, porté par le bras du robot, et qui scanne la région d'intérêt, pour enregistrer dans un repère tridimensionnel du robot les positions des points réfléchis.

Cette méthode de registration ou d'enregistrement de points est superficielle dans les deux cas. En somme, elle s'applique sur la surface externe de la peau du patient ou d'un organe préalablement préparé, notamment disséqué.

A partir de cette méthodologie, il est possible d'adapter le bras robotisé pour porter une sonde à ultrasons à son extrémité distale. En somme, l'acquisition en per-opératoire ou temps réel s'effectue par registration percutanée par ultrasons.

Il est alors possible d'effectuer des trajectoires au contact de la peau du patient et d'acquérir en percutané un nuage de points dans le repère tridimensionnel dudit bras robotisé, de manière à obtenir par exemple la forme extérieure d'un os.

En somme, la sonde à ultrasons et son système associé de traitement des données ainsi acquises sont utilisés à la manière d'un télémètre pour connaître la position des points où les ultrasons seront réfléchis, du fait d'une rupture franche de la composition des constituants biologiques, notamment la différence de densité os - muscle induisant un fort coefficient de réflexion entre les deux, tandis que ce coefficient est faible pour une combinaison de couches de peau, de muscle, de gras et d'eau. Il est ainsi possible de mesurer en percutané la position d'une surface osseuse à travers une couche de muscle et de la peau.

Selon le type d'intervention et la zone anatomique visée, ladite sonde peut émettre des ultrasons selon des fréquences d'approximativement 5 à 8 MHz (Mégahertz).

Par la suite, la forme tridimensionnelle mesurée en per-opératoire par le bras robotisé sera recalée sur la forme issue de l'image tridimensionnelle du scanner réalisé préalablement en préopératoire. A savoir que des outils de segmentation permettront préalablement d'isoler la région anatomique d'intérêt sur l'image préopératoire, notamment un os, tel une vertèbre dans l'exemple présent. On dispose ainsi d'un recalage initial servant de référence pour le suivi des mouvements.

Dans ce cas précis, ledit bras robotisé portera plusieurs émetteurs récepteurs ultrasons placés au contact de la peau, en regard de surfaces remarquables de la vertèbre, accessibles depuis une position en décubitus ventral du patient (voie postérieure). Les formes discriminantes des lames, de l'épine et des apophyses transverses de ladite vertèbre sont à même de fournir suffisamment d'informations, pour reconstruire en temps réel l'image tridimensionnelle de la vertèbre. Puis, par une mise en correspondance, de l'image tridimensionnelle ou des points remarquables, les images de la vertèbre acquises en per-opératoire sont superposées avec les images préopératoires, de manière à la situer dans le repère dudit bras robotisé.

Ainsi, un tel recalage est non invasif. En somme, le système est capable de repérer la structure anatomique interne, par exemple une vertèbre lombaire, sans dissection préalable.

Une autre solution de recalage peut utiliser une méthode bi- ou tridimensionnelle par fluoroscopie. En somme, l'acquisition en per-opératoire s'effectue par repérage de la structure interne de ladite zone anatomique par fluoroscopie. Toutefois, l'utilisation d'ultrasons présente l'avantage de s'affranchir du rayonnement nocif de la fluoroscopie.

On notera que la fluoroscopie pourra être utilisée de façon complémentaire aux ultrasons par exemple, afin d'obtenir un recalage anatomique de référence ou à des instants donnés durant l'opération, limitant ainsi le rayonnement. Ces recalages de référence et complémentaires permettront notamment de reconduire les autres recalages anatomiques effectués, pour les contrôler, mais aussi d'aider à rassembler les différents bras robotisés au sein d'un même repère tridimensionnel grâce à une mire de repérage portée par l'un des bras robotisés.

Dès lors, le système d'émission du rayonnement fluoroscopique peut équiper la table d'opération sur laquelle le patient est installé en vue de l'opération, tandis que la mire de repérage est portée par le bras robotisé.

Grâce à une caractéristique essentielle du dispositif de la présente invention, on capte par la suite les mouvements de ladite zone anatomique et on compense les déplacements dudit premier bras 3 en fonction des mouvements captés.

En somme, l'invention permet de mesurer en temps réel l'amplitude, la direction et le sens des mouvements du corps du patient, en particulier de la zone anatomique, au sein du repère dudit premier bras robotisé 3, afin de modifier ses trajectoires en conséquences, voire d'anticiper lesdits mouvements et de corriger les trajectoires à l'avance.

Avantageusement, une méthode utilisant l'invention consiste à positionner au contact de ladite zone anatomique des capteurs de détection desdits mouvements de la zone anatomique, lesdits capteurs étant solidaires de l'extrémité d'au moins un second bras 4. Selon le mode de réalisation, un tel second bras 4 peut être motorisé dans une version robotisée, à savoir qu'il est pourvu de plusieurs tronçons motorisés entre eux, à l'instar du premier bras robotisé 3.

En somme, le second bras robotisé est motorisé au niveau de ses articulations, de manière à se mouvoir et se positionner dans l'espace selon des instructions préalablement transmises auxdites motorisations.

A l'inverse, les déplacements dans l'espace de ce second bras 4 peuvent être directement connus. En somme, lorsque l'extrémité distale du second bras 4, maintenue au contact ou rendue solidaire de la zone anatomique, se déplace, alors ce déplacement se répercute aux articulations motorisées des différents tronçons qui constituent ledit second bras 4. Les mouvements des articulations permettent donc de connaître le déplacement de chaque tronçon et le mouvement dudit second bras 4 avec exactitude.

De plus, le second bras 4 étant dans un même repère ou bien un repère différent, mais dont la transposition est connue par rapport au repère du premier bras 3, l'invention prévoit de répercuter directement les déplacements dudit second bras 4 pour modifier les déplacements dudit premier bras 3.

En somme, les déplacements physiques du second bras 4 sont transmis et appliqués au premier bras 3 déjà en mouvement.

Ainsi, l' invention peut capter les mouvements internes de la zone anatomique et ses mouvements externes qui induisent les déplacements du second bras robotisé 4. Ce sont ces mouvements internes, externes et les déplacements du second bras 4 qui sont appliqués pour modifier la trajectoire du premier bras 3.

Plus particulièrement, l'invention est configurée pour détecter lesdits mouvements internes et externes et les déplacements au moyen desdits capteurs et dudit second bras et à asservir le positionnement dudit premier bras 3.

En outre, l'asservissement s'effectue par l'intermédiaire de moyens dédiés, au moins en partie constitués par des moyens de commande des déplacements dudit premier bras 3. De plus, ces moyens d'asservissement permettent de recevoir les mesures des déplacements internes et externes de la zone anatomiques, ainsi que du second bras robotisé 4, pour combiner ces mesures et les transcrire en vue d'en appliquer les résultats sur la trajectoire dudit premier bras 3.

Ainsi, l'invention fait intervenir un ou plusieurs bras secondaires 4, afin de mesurer avec exactitude les mouvements du corps 1 du patient, en vue de rectifier le positionnement dudit premier bras 3 et conserver le guidage de l'intervention prévu dans la planification préopératoire. En somme, ledit second bras 4, du fait de sa précision, offre au premier bras 3 une compensation exacte et répétable desdits mouvements.

Pour ce faire, lesdits premier 3 et second 4 bras travaillent de façon distincte ou indépendante : l'un 3 positionne l'instrument chirurgical tandis que l'autre 4 lui fournit les informations pour conserver un bon positionnement.

Dans l'exemple d'application à une vertèbre, le second bras 4 suit les mouvements de cette dernière et transmet ces changements audit premier bras 3. Comme évoqué précédemment, ces changements peuvent être internes et externes, sans induire de déplacement dudit second bras 4, mais aussi et surtout lorsque ces mouvements provoquent son déplacement.

Selon une caractéristique spécifique, lesdits premier bras robotisé 3 et le second 4 bras robotisé peuvent comprendre un même repère tridimensionnel au sein duquel ils évoluent. En d'autres termes, ledit second bras robotisé 4 est constitué de façon similaire au premier bras 3 et présente une base 40 située dans un même repère que la base dudit premier bras robotisé 3.

Dans une autre configuration, ledit second bras 4 peut évoluer au sein d'un repère distinct, mais défini par rapport au repère dudit premier bras 3, permettant au travers d'une étape de calcul, une transposition des coordonnées de l'un vers l'autre. Le second bras 4 permet donc de modifier son repère et le repère de mesure des mouvements internes et externes au cours de l'opération, tout en calculant sa liaison avec le repère du premier bras 3.

De plus, selon un mode préférentiel de réalisation, l'invention est configurée pour mesurer les efforts d'au moins un desdits bras 3,4 lors du contact avec ladite zone anatomique. En particulier, chacun des premier 3 et second 4 bras est équipé d'au moins un capteur d'effort au niveau de son extrémité distale respective 30 et 41.

Cette mesure de l'effort permet de vérifier et de configurer la force appliquée aux instruments chirurgicaux, ainsi qu'aux différents capteurs et appareils de mesure. De plus, elle permet d'offrir un mode coopératif dans lequel le praticien pourra manipuler l'extrémité 30 dudit premier bras 3 en vue d'être guidé lors de l'intervention.

Dans le cas du second bras 4, le capteur d'effort permet de maintenir une pression adaptée, assurant un bon contact des capteurs avec la zone anatomique, notamment la peau.

A ce titre, selon un premier mode de réalisation, la détection des mouvements internes de la zone anatomique peut s'effectuer par mesure percutanée au moyen de capteurs à ultrasons. Cette technique est similaire à celle évoquée précédemment pour le recalage anatomique de référence.

Dans ce cas précis et dans l'exemple préférentiel décrit, ledit bras robotisé, en particulier le second bras 4, portera plusieurs émetteurs récepteurs ultrasons placés au contact de la peau, en regard de surfaces remarquables de la vertèbre, accessibles depuis une position en décubitus ventral du patient (voie postérieure). Les formes discriminantes des lames, de l'épine et des apophyses transverses de ladite vertèbre sont à même de fournir suffisamment d'informations, pour reconstruire, en temps réel, le déplacement tridimensionnel de la vertèbre.

Il est toutefois possible de recourir à des recalages locaux de points remarquables avec les images de la vertèbre acquises en préopératoire, pour optimiser la recherche de la position exacte de la vertèbre en mouvement. Puis, par un calcul de changement de coordonnées, le recalage de référence est mis à jour afin de l'utiliser dans le repère de travail des bras robotisés 3,4.

De plus, les émetteurs récepteurs ultrasons sont placés au contact de la peau par le second bras robotisé 4 en des positions connues, permettant de suivre les mouvements externes de la zone anatomique. En somme, les coordonnées dans l'espace du point ou de la surface de contact des capteurs est connues, du fait que lesdits capteurs sont portés par le second bras robotisé 4.

Dans ce contexte, ledit second bras 4 consiste en des moyens de référencement des positions dans l'espace desdits capteurs de détection et que les moyens d'asservissement du positionnement dudit bras 3 comprennent des moyens de prise en compte du référencement desdites positions.

Cette solution présente toujours l'avantage d'être non invasive. De plus, le système est capable de repérer les mouvements de la structure interne, par exemple une vertèbre lombaire, sans dissection préalable.

Selon un autre mode de réalisation, qui ne fait pas partie de la présente invention, la détection des mouvements internes s'effectue par mesure des déplacements dans l'espace d'au moins un marqueur rendu solidaire de ladite zone anatomique. En particulier, dans l'exemple d'une vertèbre, ledit marqueur est vissé de façon invasive dans l'épine dorsale ou toutes autres zones osseuses préférentielles, en per-opératoire.

Ainsi, le marqueur et la vertèbre deviennent solidaires l'un de l'autre. De plus, la position d'implantation dudit marqueur par rapport à la vertèbre pourra être déterminée avec exactitude, sachant que la position de la vertèbre dans le repère des bras 3,4 peut déjà être connue préalablement par le recalage anatomique de référence ; cela par acquisition d'images, notamment par fluoroscopie ou ultrasons.

Une fois la position de référence déterminée, la position du marqueur dans le repère des bras 3,4 est mesurée, puis remise à jour de façon régulière ou en continu.

A ce titre, selon différents modes de réalisation, la mesure des déplacements s'effectue par émission par ledit marqueur d'un signal optique, ultrason, électromagnétique et/ou mécanique, puis réception dudit signal par lesdits capteurs réciproquement optique, ultrason, électromagnétique et/ou mécanique.

Plus particulièrement, ledit marqueur peut être directement relié auxdits capteurs sous la forme d'une liaison mécanique avec l'extrémité distale 41 du second bras 4. Dès lors, dans ce dernier cas, ladite liaison mécanique peut être prévue articulée, notamment sous forme d'un treillis géométrique. Un tel treillis offre l'avantage de démultiplier l'amplitude des mouvements mesurés, afin d'obtenir une plus grande précision de correction de la position du premier bras robotisé 3.

On notera que les corrections de cette position peuvent être opérées par rapport à l'origine du repère dudit premier bras 3, par rapport à une position initiale, ou bien par rapport à la position actuelle de son extrémité distale 30.

De plus, ledit capteur d'effort peut être réglé de manière à optimiser la mesure des mouvements perçus par cette liaison mécanique.

Ainsi, la mesure des mouvements internes et externes enregistrée par le second bras 4 est fournie audit premier bras 3, qui l'utilise pour repositionner correctement l'instrument chirurgical qu'il supporte sur la cible anatomique, en fonction de la trajectoire préalablement planifiée. De plus, cette mesure permet de corriger en temps réel et de mettre à jour automatiquement le recalage anatomique effectué, même au cours du déplacement de l'instrument chirurgical.

Selon une caractéristique additionnelle, les mouvements anatomiques détectés peuvent être anticipés, en particulier par des simulations de mouvements réguliers de la zone anatomique. Dans l'exemple du rachis lombaire, les mouvements respiratoires peuvent être connus, notamment leur période et l'amplitude, pour être pris en compte dans la modification de la trajectoire.

Comme évoqué précédemment, l'invention concerne un dispositif 2 robotique de positionnement d'instrument chirurgical par rapport au corps 1 d'un patient.

Un tel dispositif 2 s'apparente à une plateforme 5 robotisée et comprend de ce fait un premier bras robotisé 3 équipé à une extrémité 30 de moyens de solidarisation avec au moins un instrument chirurgical ; des moyens de recalage anatomique dudit premier bras 3 par rapport audit corps 1, ledit recalage s'effectuant par recalage d'une image d'une zone de l'anatomie dudit patient acquise en per-opératoire par rapport à une imagerie acquise préalablement ; des moyens de détection des mouvements internes de ladite zone anatomique et des moyens de compensation en temps réel des déplacements dudit premier bras 3 en fonction desdits mouvements détectés.

Il comprend au moins un second bras robotisé 4 équipé à une extrémité 4 équipé à une extrémité desdits capteurs de détection desdits mouvements internes de la zone anatomique ; des moyens d'asservissement du positionnement dudit premier bras 3 par rapport, d'une part, auxdits mouvements internes captés et, d'autre part, par rapport aux mouvements externes induits audit second bras 4.

Ainsi, la présente invention permet d'assurer la précision robotisée lors du positionnement d'un instrument chirurgical au niveau d'une zone anatomique, tout en s'adaptant aux mouvements du corps du patient.

En outre, l'utilisation d'un second bras robotisé permet d'obtenir des données concernant les mouvements externes, sans faire intervenir de systèmes supplémentaire ou complémentaire de détection de la position des capteurs.

## Revendications

1. Dispositif d'assistance chirurgicale robotique automatisé (2) destiné à positionner au moins un instrument chirurgical par rapport à une région anatomique du corps (1) d'un patient, comprenant :
un premier bras robotisé (3) ;
le au moins un instrument chirurgical disposé à une extrémité distale (30) du premier bras robotisé ;
un capteur configuré pour détecter un mouvement dans la région anatomique par rapport au au moins un instrument chirurgical, disposé à une extrémité distale d'un deuxième bras robotisé ; et
un dispositif de commande robotique configuré pour :
commander automatiquement le premier bras robotisé (3) comprenant le au moins un instrument chirurgical disposé à une extrémité distale du premier bras robotisé (3) afin de positionner le au moins un instrument chirurgical le long d'une trajectoire prévue par rapport à la région anatomique d'après une image préopératoire de la région anatomique ;
déterminer une indication du mouvement dans la région anatomique par rapport au au moins un instrument chirurgical, à partir de mesures du capteur ainsi que de mesures des déplacements du deuxième bras robotisé ; et
commander automatiquement le premier bras robotisé (3), en réponse à la détermination de l'indication, afin de compenser le mouvement de la région anatomique en maintenant la position du au moins un instrument chirurgical le long de la trajectoire prévue.

2. Dispositif d'assistance chirurgicale robotique automatisé selon la revendication 1, dans lequel le capteur comprend un capteur de force adapté pour être en contact avec la région anatomique, et comprenant en outre la détection de l'indication de mouvement à l'aide du capteur de force.

3. Dispositif d'assistance chirurgicale robotique automatisé selon la revendication 1, dans lequel le capteur comprend au moins l'un parmi le groupe comprenant un capteur à ultrasons, un capteur optique, un capteur électromagnétique, ou un capteur mécanique.

4. Dispositif d'assistance chirurgicale robotique automatisé selon la revendication 1, dans lequel le deuxième bras robotisé est adapté pour être en contact avec la région anatomique et se déplacer en réponse à un mouvement de la région anatomique.

5. Dispositif d'assistance chirurgicale robotique automatisé selon la revendication 4, dans lequel le mouvement du deuxième bras robotisé (4) peut être causé par le mouvement de la région anatomique, notamment par la force directe exercée par la région anatomique, ou
dans lequel le capteur est un capteur à ultrasons et le dispositif de commande robotique est en outre configuré pour commander le deuxième bras robotisé afin d'entraîner le maintien d'une distance entre le deuxième bras robotisé et la région anatomique, en fonction du résultat d'une détection du capteur à ultrasons.

6. Dispositif d'assistance chirurgicale robotique automatisé selon la revendication 2, dans lequel le dispositif de commande robotique est en outre configuré pour :
détecter, à l'aide du capteur de force, un mouvement d'entrée par un chirurgien en mode coopératif ; et
commander le premier bras robotisé (3) en fonction du mouvement d'entrée.

7. Dispositif d'assistance chirurgicale robotique automatisé selon la revendication 1, dans lequel le dispositif de commande robotique est en outre configuré pour commander automatiquement le premier bras robotisé (3) afin de réaliser une partie d'une intervention chirurgicale sans contact direct sur le premier bras robotisé (3) par un chirurgien.

8. Dispositif d'assistance chirurgicale robotique automatisé selon la revendication 7, dans lequel la partie d'une intervention chirurgicale est une incision chirurgicale percutanée.

9. Dispositif d'assistance chirurgicale robotique automatisé selon l'une quelconque des revendications 1 à 8, dans lequel le dispositif de commande robotique est en outre configuré pour commander automatiquement le premier bras robotisé (3) afin de maintenir une position et une orientation du premier bras robotisé par rapport à la région anatomique.

## Patentansprüche

1. Automatisierte robotergestützte chirurgische Hilfsvorrichtung (2), das zum Positionieren mindestens eines chirurgischen Instruments in Bezug auf einen anatomischen Bereich des Körpers (1) eines Patienten bestimmt ist, umfassend:
einen ersten vollautomatisierten Arm (3);
das mindestens eine chirurgische Instrument, das an einem distalen Ende (30) des ersten vollautomatisierten Arms angeordnet ist;
einen Sensor, der konfiguriert ist, um eine Bewegung in dem anatomischen Bereich in Bezug auf das mindestens eine chirurgische Instrument zu erfassen, der an einem distalen Ende eines zweiten vollautomatisierten Arms angeordnet ist; und
eine robotergestützte Steuervorrichtung, die konfiguriert ist, um:
den ersten vollautomatisierten Arm (3), der das mindestens eine chirurgische Instrument umfasst, das an einem distalen Ende des ersten vollautomatisierten Arms (3) angeordnet ist, automatisch zu steuern, um das mindestens eine chirurgische Instrument entlang einer vorgesehenen Bahn in Bezug auf den anatomischen Bereich auf Grundlage eines präoperativen Bildes des anatomischen Bereichs zu positionieren;
Bestimmen einer Angabe der Bewegung in dem anatomischen Bereich in Bezug auf das mindestens eine chirurgische Instrument aus Messungen des Sensors sowie aus Messungen der Versetzungen des zweiten vollautomatisierten Arms; und
automatisch den ersten vollautomatisierten Arms (3) als Reaktion auf die Bestimmung der Anzeige zu steuern, um die Bewegung des anatomischen Bereichs zu kompensieren, indem die Position des mindestens einen chirurgischen Instruments entlang der vorgesehenen Bahn beibehalten wird.

2. Automatisierte robotergestützte chirurgische Hilfsvorrichtung nach Anspruch 1, wobei der Sensor einen Kraftsensor umfasst, der angepasst ist, mit dem anatomischen Bereich in Kontakt zu sein, und ferner das Erfassen der Bewegungsanzeige mit Hilfe des Kraftsensors umfasst.

3. Automatisierte robotergestützte chirurgische Hilfsvorrichtung nach Anspruch 1, wobei der Sensor mindestens einen aus der Gruppe umfasst, die einen Ultraschallsensor, einen optischen Sensor, einen elektromagnetischen Sensor oder einen mechanischen Sensor umfasst.

4. Automatisierte robotergestützte chirurgische Hilfsvorrichtung nach Anspruch 1, wobei der zweite vollautomatisierte Arm angepasst ist, um mit dem anatomischen Bereich in Kontakt zu sein und sich als Reaktion auf eine Bewegung des anatomischen Bereichs zu bewegen.

5. Automatisierte robotergestützte chirurgische Hilfsvorrichtung nach Anspruch 4, wobei die Bewegung des zweiten vollautomatisierten Arms (4) durch die Bewegung des anatomischen Bereichs auslösbar ist, insbesondere durch die direkte Kraft, die von dem anatomischen Bereich ausgeübt wird, oder
wobei der Sensor ein Ultraschallsensor ist und die robotergestützte Steuervorrichtung ferner konfiguriert ist, um den zweiten vollautomatisierten Arm zu steuern, um ein Aufrechterhalten eines Abstands zwischen dem zweiten vollautomatisierten Arm und dem anatomischen Bereich in Abhängigkeit von dem Ergebnis einer Erfassung des Ultraschallsensors zu bewirken.

6. Automatisierte robotergestützte chirurgische Hilfsvorrichtung nach Anspruch 2, wobei die robotergestützte Steuervorrichtung ferner konfiguriert ist, um:
den Kraftsensor mit Hilfe einer Eintrittsbewegung durch einen Chirurgen im kooperativen Modus zu erfassen, und
den ersten vollautomatisierten Arm (3) in Abhängigkeit von der Eintrittsbewegung zu steuern.

7. Automatisierte robotergestützte chirurgische Hilfsvorrichtung nach Anspruch 1, wobei die robotergestützte Steuervorrichtung ferner konfiguriert ist, um den ersten vollautomatisierten Arm (3) automatisch zu steuern, um einen Teil eines chirurgischen Eingriffs ohne direkten Kontakt mit dem ersten vollautomatisierten Arm (3) durch einen Chirurgen durchzuführen.

8. Automatisierte robotergestützte chirurgische Hilfsvorrichtung nach Anspruch 7, wobei der Teil eines chirurgischen Eingriffs ein perkutaner chirurgischer Schnitt ist.

9. Automatisierte robotergestützte chirurgische Hilfsvorrichtung nach einem der Ansprüche 1 bis 8, wobei die robotergestützte Steuervorrichtung ferner konfiguriert ist, um den ersten vollautomatisierten Arm (3) automatisch zu steuern, um eine Position und eine Ausrichtung des ersten vollautomatisierten Arms in Bezug auf den anatomischen Bereich aufrechtzuerhalten.

## Claims

1. Automated robotic surgical assistance device (2) intended to position at least one surgical instrument relative to an anatomical region of the body (1) of a patient, comprising:
- a first robotic arm (3);
with the at least one surgical instrument being disposed at a distal end (30) of the first robotic arm;
- a sensor configured to detect a movement in the anatomical region relative to at least one surgical instrument disposed at a distal end of a second robotic arm; and
- a robotic control device configured for:
- automatically controlling the first robotic arm (3) comprising the at least one surgical instrument disposed at a distal end of the first robotic arm (3) in order to position the at least one surgical instrument along an intended trajectory relative to the anatomical region based on a preoperative image of the anatomical region;
- determining an indication of the movement in the anatomical region relative to the at least one surgical instrument on the basis of measurements of the sensor and of measurements of the movements of the second robotic arm; and
- automatically controlling the first robotic arm (3), in response to determining the indication, in order to compensate for the movement of the anatomical region by maintaining the position of the at least one surgical instrument along the intended trajectory.

2. Automated robotic surgical assistance device according to Claim 1, wherein the sensor comprises a force sensor adapted to be in contact with the anatomical region, and further comprising detecting the indication of movement using the force sensor.

3. Automated robotic surgical assistance device according to Claim 1, wherein the sensor comprises at least one among the group comprising an ultrasound sensor, an optical sensor, an electromagnetic sensor, or a mechanical sensor.

4. Automated robotic surgical assistance device according to Claim 1, wherein the second robotic arm is adapted to be in contact with the anatomical region and to move in response to movement of the anatomical region.

5. Automated robotic surgical assistance device according to Claim 4, wherein the movement of the second robotic arm (4) can be caused by the movement of the anatomical region, notably by the direct force exerted by the anatomical region, or
wherein the sensor is an ultrasound sensor and the robotic control device is further configured to control the second robotic arm in order to maintain a distance between the second robotic arm and the anatomical region, as a function of the result of a detection by the ultrasound sensor.

6. Automated robotic surgical assistance device according to Claim 2, wherein the robotic control device is further configured to:
- detect, using the force sensor, an entry movement by a surgeon in a cooperative mode; and
- control the first robotic arm (3) as a function of the entry movement.

7. Automated robotic surgical assistance device according to Claim 1, wherein the robotic control device is further configured to automatically control the first robotic arm (3) in order to perform part of a surgical procedure without direct contact on the first robotic arm (3) by a surgeon.

8. Automated robotic surgical assistance device according to Claim 7, wherein the part of a surgical procedure is a percutaneous surgical incision.

9. Automated robotic surgical assistance device according to any one of Claims 1 to 8, wherein the robotic control device is further configured to automatically control the first robotic arm (3) in order to maintain a position and an orientation of the first robotic arm relative to the anatomical region.
